# EUROPÄISCHE PATENTSCHRIFT

(11) **EP 2 305 790 B1**
(45) Veröffentlichungstag und Bekanntmachung des Hinweises auf die Patenterteilung: **05.09.2012**
(21) Anmeldenummer: 10008549.7
(22) Anmeldetag: 16.08.2010
(51) Int. Cl.: G01N 1/20

(54) **Vorrichtung zur Probenahme**
Sampling device
Dispositif destiné au prélèvement d'échantillons

(30) Priorität: 01.10.2009 DE 102009043699
(43) Veröffentlichungstag der Anmeldung: 06.04.2011
(73) Patentinhaber: Infors AG, 4103 Bottmingen (CH)
(72) Erfinder: Brücher, Daniel, 40593 Düsseldorf (DE)
(74) Vertreter: Säger, Manfred

(56) Entgegenhaltungen:
- DE-A1- 2 650 405
- DE-B3-102004 045 785
- US-A- 5 624 638
- US-A1- 2002 129 858
- US-A1- 2005 214 927
- US-A1- 2007 193 376

## Beschreibung

Die Erfindung betrifft eine gattungsgemässe Vorrichtung zum Nehmen einer Probe nach dem Oberbegriff des Hauptanspruchs, nämlich eine Vorrichtung zur Probenahme von Kulturlösungen wie Mikroorganismen (z.B. Bakterien, Hefen, Pilze) oder Zellen (z.B. Säuger- und Insektenzellen) über einen Verbindungsschlauch aus vorzugsweise autoklavierbaren Kulturgefäßen wie Bioreaktoren oder Einweg-Kultivierungssysteme wie Kunststoffbeutel zur Kultivierung von Mikroorganismen oder Zellen mit einer die Probe aufnehmenden sterilen Spritze mit sogenanntem Luer-Lock-Konus und mit einem bei deren Aufsetzen selbsttätig öffenenden und bei Abnehmen selbsttätig schliessenden Automatikventil, auch Injection Site genannt.

Kultivierungen von Bakterien, Hefen, Pilzen und tierischen Zellen sowie Säugerzellen werden im kleinen Maßstab unter anderem in autoklavierbaren Bioreaktoren und in Einweg-Kunststoffbeuteln (DE 10 2004 045 916 A1) durchgeführt. Während der Kultivierung müssen Proben der Kultur entnommen werden, um z.B. Wachstumsparameter bestimmen zu können. Bei der Entnahme von Proben muss sichergestellt sein, dass eine Kontamination des Kulturgefäßes mit Fremdkeimen vermieden wird.

Eine solche gattungsgemässe Vorichtung ist bekannt. Diese bislang existierende steriltechnische Lösung setzt das Automatikventil ein, das durch Aufsetzen der sterilen Spritze mit Luer-Konus automatisch öffnet und bei Abnehmen der Spritze selbsttätig schließt. Solche Automatikventile ("Injection site") sind in der Medizintechnik für Zugabe oder Entnahme von Flüssigkeiten üblich und etabliert, z.B. für Verwendung an Infusionsbestecken, Blutbeutel etc. und sind von verschiedenen Herstellern auf dem Markt erhältlich.

Die gattungsgemässe Vorrichtung ist jedoch in mancherlei Hinsicht nachteilig. So ermöglicht sie nämlich sowohl die Zugabe als auch Entnahme von Flüssigkeiten, was beim Einsatz für die Probenahme nicht erwünscht ist, da das ungewollte Zuführen von Flüssigkeiten das Kulturgefäß kontaminieren könnte. Somit besteht beim Einsatz für die Probenahme die Gefahr einer Fehlbedienung. Nach der Probenahme bleiben das Automatikventil und der damit und dem Kulturgefäss verbundene Verbindungsschlauch mit einer die Probe bildenden Kulturlösung gefüllt, was zur Folge hat, dass vor der nächsten Probenahme das System "gespült" werden muss. Dabei wird zunächst eine bestimmte Menge an Flüssigkeit entnommen und verworfen, bevor die eigentliche Probe genommen werden kann. Dies kann bei Kulturgefäßen mit kleinen Volumina ein Problem bilden, weil durch das Spülen zu viel Volumen verloren geht. Insgesamt stellt sich die bekannte Vorrichtung als nur einegschränkt brauchbar dar.

US2007/0193376 offenbart eine Vorrichtung zur sterilen Probennahme von Kulturen. Die Probenahme-Vorrichtung weißt eine Luer-Lock-Konus-Verbindung auf. Die Sterilität wird dadurch gewährt, dass eine durch eine Nadel punktierbare Bohrung vorgesehen ist.

EP1719997 offenbart eine Probenentnahmevorrichtung. Zur Probeentnahme kann die Probeentnahmespritze direkt oder durch Verbindungsleitungen am Fermenter angebracht sein. Der Anschluss der Probeentnahmespritze kann mit einem Rückschlagventil ausgestattet sein.

Der Erfindung liegt deshalb die Aufgabe zugrunde, eine gattungsgemässe Vorrichtung in Funktion und Wirkunsweise besser sowie zugleich einfacher auszugestalten.

Diese Aufgabe wird bei einer gattungsgemässen Vorrichtung nach dem Oberbegriff des Hauptanspruchs erfindungsgemäss durch dessen kennzeichnende Merkmale also dadurch gelöst, dass zwischen dem Automatikventil und der Verbindung ein zu diesem hin schliessendes erstes Rückschlagventil vorgesehen ist.

Durch das erste Rückschlagventile ist nach Lehre der Erfindung eine Probenahme mit der sterilen Spritze über die Verbindung, nicht jedoch das Zuführen von Flüssigkeiten in umgekehrter Fliessrichtung möglich, womit erstmals gegeüber dem Stand der Technik eine sterile Probenahme ohne die Verunreingung der Kultur möglich ist.

Wenn in vorteilhafter Weiterbildung der Erfindung zwischen dem Automatikventil und der Verbindung ein diese über eine Durchgangsleitung verbindendes T-Stück mit einer Stichleitung angeordnet ist, wobei in der Durchgangsleitung das zu dem T-Stück hin schliessende erste Rückschlagventil vorgesehen ist und wobei in der Stichleitung ein zu dem T-Stück hin öffnendes zweites Rückschlagventil angeordnet ist.

Dabei sperrt das zweite Rückschlagventil nicht nur das Eindringen von Kulturlösung in die Stichleitung selbstätig; ausserdem kann nach der Probenahme die Vorrichtunmg mit steriler Luft praktisch vollständig entleert werden, so dass es somit quasi totvolumenfrei ist. Schliesslich erlaubt die erfindungsgemässe Vorrichtung lediglich das Einbringen von Luft über den vorzugsweise vorgesehehen Sterilfilter, jedoch nicht eine Entnahme der Kulturlösung, die den Sterilfilter beschädigen würde. Durch die beiden Rückschlagventile ist also nach Lehre der Erfindung eine Probenahme mit der sterilen Spritze über die Durchgangsleitung, nicht jedoch das Zuführen von Flüssigkeiten in umgekehrter Fliessrichtung möglich.

Die Probenahme erfolgt dabei so, dass die sterile Spritze auf das Automatikventil aufgesetzt und ihr Kolben herausgezogen wird, um die Probe zu entnehmen. Danach wird die sterile Spritze von dem Automatikventil gelöst. Wegen des in der Stichleitung zu dem T-Stück hin öffnenden zweiten Rückschlagventils kann die Probe nicht in die Stichleitung gelangen. Danach wird der Kolben einer am Ende der Stichleitung vorgesehenen -auch unsterilenzweiten Spritze herausgezogen, um diese mit Luft zu füllen, und nach dem Anschliessen an die einen Luftfilter aufweisende Stichleitung so oft herausgedrückt, bis die noch in der zwischen dem T-Stück und der Verbindung zu dem Kulturgefäss befindliche Kulturlösung wieder in dieses zurückverdrängt ist.

Die neue Probenahmevorrichtung nach der Erfindung bietet also auf überraschend einfache Weise die aseptische Probenahme in einer sehr einfacher Art und Weise. Es bietet die Möglichkeit, nach der Probenahme die Kulturlösung in der Vorrichtung mit steriler Luft zu verdrängen, wodurch das bisher erforderliche nachträgliche Spülen der Probenahme und Verwerfen der Kulturlösung nicht mehr notwendig ist. Dies ist besonders für kleine Kulturgefäße von grosser Wichtigkeit, wenn wegen der Probenahme das im Kulturgefäß verbleibende Volumen unter einen kritischen Wert fallen könnte. Auch sind Fehlbedienungen konstruktiv ausgeschlossen, weil die Fließrichtungen durch die Rückschlagventile eindeutig festgelegt sind. Dadurch ist auch eine versehentliche Kontaminierung des Kulturgefäßes über die Probenahme nicht möglich. Auch das sterile Weiterverarbeiten der Proben ist möglich. Das Totvolumen kann auf weniger als 0,1 ml reduziert werden, was einen vernachlässigbaren kleinen Wert darstellt. Schliesslich ist es von grossem Vorteil, dass das System mehrfach autoklaviert werden kann. Schliesslich ist das ungewollte Einbringen von Kulturlösung in den Luftfilter durch die Erfindung konstruktionsbedingt nicht möglich. Letzlich ist das manuelle Absperren der Probenahme nicht mehr erforderlich; dies übernimmt das Automatikventil. Es ist aber nach Lehre der Erfindung auch möglich, ohne die beiden Rückschlagventile dann mit weniger Komfort bei der Messung auszukommen, wenn die selbsttätig arbeitenden Rückschlagventile durch manuelle Absperrventile ersetzt werden oder bei der Probenahme zumindest die Stichleitung manuell z.B. durch ein Absperrventil oder eine Schlauchklemme abgesperrt wird.

Mit besonderem Vorteil besteht bei der erfindungsgemässen Vorrichtung die sterile Spritze mit dem Luer-Lock-Konus, die zweite Spritze, das Automatikventil, das T-Stück, die Stichleitung, das erste Rückschlagventil, das zweite Rückschlagventil, das Schlauchstück, der Luftfilter und/oder der Verbindungsschlauch als System aus an sich bekannten, miteinander kombinierbaren handelsüblichen Bauteilen und wird als ggf. teilweise zusammengabautes Set geliefert, so das diese in einfachster Weise und billig sowie einfach zu der Vorrichtung aufgebaut werden können.

Weitere zweckmässige Ausgestaltungen und Weiterbildungen der Erfindung sind in den restlichen Unteransprüchen gekennzeichnet.

Ein Ausführungsbeispiel wird nachfolgen unter Bezugnahme auf die Zeichnung näher erläutert. In dieser zeigt:
- Figur 1: die Vorrichtung als Sprengbild und
- Figur 2: die Vorrichtung in zusammengebautem Zustand.

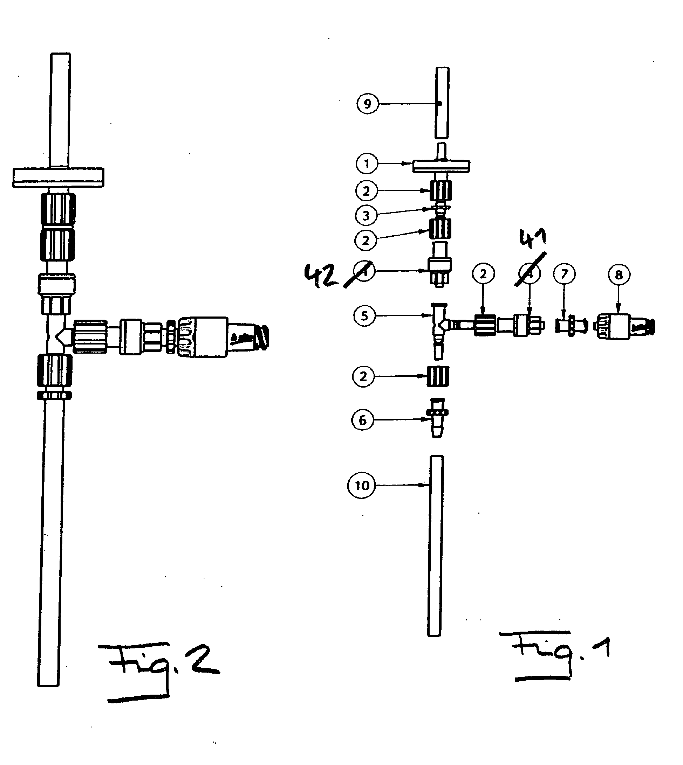

Die in Figur 1 dargestellte Vorrichtung dient zur Probenahme von Kulturen wie Mikroorganismen oder Zellen über eine als Verbindungsschlauch ausgebildeten Verbindung 10 aus -nicht gezeigten- autoklavierbaren Kulturgefäßen mit einer die eigentliche Probe aufnehmenden sterilen Spritze mit sogenanntem Luer-Lock-Konus über ein bei deren Aufsetzen selbsttätig öffenenden und bei Abnehmen selbsttätig schliessenden Automatikventil 8.

Zwischen diesem Automatikventil 8 und der Verbindung (10) ist ein diese über eine Durchgangsleitung verbindendes T-Stück 5 mit einer einen Luftfilter 1 aufweisenden Stichleitung angeordnet, an deren Ende eine zweite Spritze über ein Schlauchstück 9 anschliessbar ist. In der Durchgangsleitung ist ein zu dem T-Stück 5 hin schliessendes erstes Rückschlagventil 41 vorgesehen und in der Stichleitung ist ein zu dem T-Stück hin öffnendes zweites Rückschlagventil 42 angeordnet.

Bei der erfindungsgemässen Vorrichtung sind vorzugsweise die sterile Spritze mit dem Luer-Lock-Konus, die zweite Spritze, das Automatikventil 8, das T-Stück 5, die Stichleitung, das erste Rückschlagventil 41, das zweite Rückschlagventil 42, das Schlauchstück 9, der Luftfilter 1 und/oder der Verbindungsschlauch 10 als an sich bekannte, miteinander kombinierbare handelsübliche Bauteile ausgebildet, die als Set geliefert werden, weil sie in einfacher Weise zu der Vorrichtung zusammengebaut werden können.

Der Luftfilter 1 wird dabei auf einen Fingerring 2 mit Luer-Lock Arretierung eingerastet, der an Luer-Verbinder 3 männlich/männlich ageschlossen ist, der selbst wieder mit einem Fingerring 2 zum Anschluss an das zweite Rückschlagventil 42 verbunden ist. Mit 7 ist ein Luer-Verbinder weiblich/weiblich und mit 6 ein Luer-Verbinder weiblich/Schlauchnippel bezeichnet.

Die Probenahme erfolgt dabei so, dass die sterile Spritze auf das Automatikventil 8 aufgesetzt und ihr Kolben herausgezogen wird, um die Probe zu entnehmen. Danach wird die sterile Spritze von dem Automatikventil 8 gelöst. Wegen des in der Stichleitung zu dem T-Stück 5 hin öffnenden zweiten Rückschlagventils 42 kann die Probe nicht in die Stichleitung gelangen. Danach wird der Kolben der am Ende der Stichleitung vorgesehenen -auch unsterilen- zweiten Spritze herausgezogen, um diese mit Luft zu füllen, und nach dem Anschliessen an die den Luftfilter 1 aufweisende Stichleitung so oft herausgedrückt, bis die noch in der zwischen dem T-Stück 5 und der Verbindung 10 zu dem Kulturgefäss befindliche Kulturlösung wieder in dieses zurückzuverdrängt ist.

## Patentansprüche

1. Vorrichtung zur Probenahme von Kulturen wie Mikroorganismen oder Zellen über eine Verbindung (10) aus autoklavierbaren Kulturgefäßen mit einer die Probe aufnehmenden sterilen Spritze mit sogenanntem Luer-Lock-Konus und mit einem bei deren Aufsetzen selbsttätig öffenenden und bei Abnehmen selbsttätig schliessenden Automatikventil (8), **dadurch gekennzeichnet, dass** zwischen dem Automatikventil (8) und der Verbindung (10) ein zu diesem (8) hin schliessendes erstes Rückschlagventil (41) vorgesehen ist.

2. Vorrichtung nach Anspruch 1, **dadurch gekennzeichnet, dass** zwischen dem Automatikventil (8) und der Verbindung (10) ein diese über eine Durchgangsleitung verbindendes T-Stück (5) mit einer Stichleitung angeordnet ist, dass in der Durchgangsleitung das zu dem T-Stück (5) hin schliessendes erstes Rückschlagventil (41) vorgesehen ist und dass in der Stichleitung ein zu dem T-Stück hin öffnendes zweites Rückschlagventil (42) angeordnet ist.

3. Vorrichtung nach einem der vorangehenden Ansprüche, **dadurch gekennzeichnet, dass** in der Stichleitung ein Luftfilter (1) angeordnet ist.

4. Vorrichtung nach einem der vorangehenden Ansprüche, **dadurch gekennzeichnet, dass** am Ende der Stichleitung eine zweite Spritze vorgesehen ist.

5. Vorrichtung nach Anspruch 4, **dadurch gekennzeichnet, dass** am Ende der Stichleitung die zweite Spritze über ein Schlauchstück (9) anschliessbar ist.

6. Vorrichtung nach Anspruch 1, **dadurch gekennzeichnet, dass** die Verbindung (10) als Verbindungsschlauch ausgebildet ist.

## Claims

1. A sampling device for sampling cultures, such as microorganisms or cells from autoclavable culture dishes via a connection (10), with a sterile syringe receiving the sample with a so-called Luer-lock cone, and with an automatic valve (8) opening automatically upon attachment and closing automatically upon removal,
**characterised in that**
between the automatic valve (8) and the connection (10), a first non-return valve (41) closing towards the former (8) is provided.

2. The device according to claim 1,
**characterised in that**,
between the automatic valve (8) and the connection (10), a T-piece (5) is arranged connecting the latter via a continuous line to a branch line
that the first non-return valve (41) closing towards the T-piece (5) is provided in the continuous line and
that a second non-return valve (42) opening towards the T-piece is arranged in the branch line.

3. The device according to any one of the preceding claims,
**characterised in that**
an air filter (1) is arranged in the branch line.

4. The device according to any one of the preceding claims,
**characterised in that**
a second syringe is provided at the end of the branch line.

5. The device according to claim 4,
**characterised in that**
the second syringe can be connected to the end of the branch line via a tubing portion (9).

6. The device according to claim 1,
**characterised in that**
the connection (10) is embodied as a connecting tube.

## Revendications

1. Dispositif destiné au prélèvement d'échantillons de cultures telles que des microorganismes ou des cellules par l'intermédiaire d'un raccordement (10) constitué de récipients de culture pouvant être autoclavés, au moyen d'une seringue stérile recevant l'échantillon, ladite seringue étant dotée d'un cône dit Luer-Lock et d'une soupape automatique (8) s'ouvrant automatiquement lorsqu'elle est posée et se fermant automatiquement lorsqu'elle est retirée, **caractérisé en ce qu'**un premier clapet anti-retour (41) se fermant en direction de la soupape automatique (8) est disposé entre ladite soupape automatique (8) et le raccordement (10).

2. Dispositif selon la revendication 1, **caractérisé en ce qu'**un raccord en T (5) reliant la soupape automatique (8) et le raccordement (10) et doté d'un piquage est disposé entre ces derniers, **en ce que** le premier clapet anti-retour (41) se fermant en direction du raccord en T (5) est disposé dans la conduite de passage et **en ce qu'**un second clapet anti-retour (42) s'ouvrant en direction du raccord en T est disposé dans le piquage.

3. Dispositif selon l'une quelconque des revendications précédentes, **caractérisé en ce qu'**un filtre à air (1) est disposé dans le piquage.

4. Dispositif selon l'une quelconque des revendications précédentes, **caractérisé en ce qu'**une seconde seringue est disposée à l'extrémité du piquage.

5. Dispositif selon la revendication 4, **caractérisé en ce que** la seconde seringue peut être raccordée à l'extrémité du piquage par un raccord en T (9).

6. Dispositif selon la revendication 1, **caractérisé en ce que** le raccordement (10) est réalisé comme un flexible de raccordement.
